# EUROPEAN PATENT SPECIFICATION

(11) **EP 3 036 370 B1**
(45) Date of publication and mention of the grant of the patent: **21.11.2018**
(21) Application number: 14837717.9
(22) Date of filing: 20.08.2014
(51) Int. Cl.: D21D 1/00, D21G 9/00

(54) **A METHOD AND A SYSTEM FOR CONTROLLING A REFINER FOR REFINING A PRODUCT CONTAINING CELLULOSE AND READABLE INFORMATION ABOUT A QUALITY INDEX OF THE PRODUCT**
VERFAHREN UND SYSTEM ZUR STEUERUNG EINES REFINERS ZUM MAHLEN EINES PRODUKTS, DAS ZELLULOSE UND LESBARE INFORMATION BETREFFEND EINES QUALITÄTSINDEX DES PRODUKTS ENTHÄLT
PROCÉDÉ ET SYSTÈME DE COMMANDE D'UN RAFFINEUR POUR RAFFINER UN PRODUIT CONTENANT DE LA CELLULOSE ET UNE INFORMATION LISIBLE CONCERNANT UN INDICE DE QUALITÉ DU PRODUIT

(30) Priority: 20.08.2013 FI 20135850
(43) Date of publication of application: 29.06.2016
(73) Proprietor: Metsä Fibre Oy, 02020 Metsä (FI)
(72) Inventor: POUKKA, Outi, FI-02020 Metsä (FI); PEKURI, Esko, FI-02020 Metsä (FI)
(74) Representative: Seppo Laine Oy
(86) International application number: PCT/FI2014/050641
(87) International publication number: WO 2015/025084

(56) References cited:
- US-A- 3 568 939
- US-A- 6 024 309
- US-A1- 2010 121 473
- US-A1- 2011 304 513
- US-A1- 2011 315 777

## Description

### Background of the Invention

### Field of the Invention

The present invention relates to a method for controlling a refiner for refining a product containing cellulose, such as pulp.

### Description of Related Art

In the pulp industry business, the logistics systems must identify and track cellulose products that are leaving the mill to ensure it is sent to the right customer at the right time. Until now, this has required visual inspection of pulp unit labels or scanning of bar codes, providing merely a means for production lot identification. In many applications where remote and fast identification is needed for identification, like in goods supply chains, RFID technology is being deployed.

Pulp units have unique requirements also in terms of quality information. Usually the pulp is pulped in water at the customer's paper factory, it is refined, its properties are analyzed and chemicals are added, as well as other process steps being taken. According to traditional methods of quality control, refining results of the pulp is measured once or twice a week. Statistically reliable information about the pulp can thus be obtained only months after it has been produced.

Traditional methods of assessing pulp quality, which is reflected in the runnability of the paper machines, are inexact, they are sensitive to measuring conditions (e.g. to seasonal variations and humidity), they require time-consuming analysis and they are generally providing only isolated analysis points which means that they do not give representative data on the whole pulp unit. Thus it is essential that the pulp units received at the paper mill are of even and predictable quality. The more uniform in quality the pulp is, and the more accurate quality information about the pulp unit is known, the easier it is for the paper manufacturer to optimize paper manufacturing, to adapt the operation of the paper machine to the actual pulp quality and, if so desired, to tailor the paper product to final specifications.

In the co-pending International Patent Application PCT/FI2013/050732 filed by the present applicant is disclosed a RFID-based solution for marking a cellulose product with quality information. The solution is based on normalizing measured parameters in each pulp preparation process and forming a contribution of each process step to an overall yield function. The contribution of each process step to the yield function adds to a quality index of the product. A recordable and remotely readable passive RFID tag, attached to e.g. a pulp unit, carries pulp unit identification and quality index information. An RFID tag with the size of a grain of sand and provided on a biodegradable paper substrate is capable of carrying up to 32 kbytes of data. Information about the pulp unit is saved in the pulp mill's reporting system, providing tracking and other logistic support information during the travel from the pulp mill to the client's paper factory.

Precise knowledge of the pulp quality in an identified pulp unit provides a unique opportunity for customers to optimise their own production. Accurate quality reporting advises the customer that a pulp lot is at the top end of the quality spectrum, meaning that it is possible to optimize or minimize the amount of the pulp or the chemical consumption. In fact, many times it is of key importance for the paper or board manufacturer to find the right amount of pulp for the corresponding paper or board or tissue product having the predetermined properties.

The main process parameters to be considered in a paper factory when refining the pulp into fibers and conveying them to the wet end of a paper machine are:
- specific energy consumption of the mill, which is the main control parameter for the refining or milling process automation. In a typical system, the specific energy consumption of the mill is kept constant
- pulp quality information,
- refining consistency (thickness) of the slurry at the mill,
- specific edge (or cutter) load at the blades of the mill (containing information about the knives, refining consistency and flow),
- Flow Indicator Control (FIC) of the refined fibers, and
- temperature.
Of these parameters, the quality index disclosed in the aforementioned document PCT/FI2013/05732 brings a crucial advantage to the quality information, as it allows on-line determination of a cellulose or pulp quality index at the pulp factory, which is then usable immediately at the paper mill. Quality information is typically obtained with intervals of ten minutes, amounting to about 1000 values a week. The advantages include availability of reliable information about possible quality variation, the quality index may replace tests for quality control purposes, and may contribute to reduced electric power consumption in the refining process, when it is known that a pulp lot has good furnishing properties.
However, even if pulp quality information is very useful in a papermaking process, there is still a lot of parameter settings and tuning to be done to achieve a desired quality of paper. Very few criteria are available for on-line control of the refining of pulp. The most known is the Canadian Standard Freeness (CSF) number. CSF is a quantitative measure of the surface area of a pulp, which again corresponds to the level of disintegration and beating of the pulp.

In modern paper mills, the CSF number is measured at the output of the refining stage, and is used as target value according to which the specific energy consumption of the mill is altered. Alternatively, the specific energy consumption of the mill is kept constant and the CSF value may then vary somewhat. A smaller CSF value means the pulp has a higher surface area (and degree of beating), and vice versa. It is usually in the interest of the manufacturer to keep the CSF value constant to ensure a constant quality of the slurry consisting of shredded and refined pulp fiber, water and chemicals. This is then done by adjusting the electrical power fed to the mill engines, to adjust the degree of beating of the pulp.

In the context of this description, a mill can mean a pulp or cellulose product manufacturing plant, or a paper manufacturing plant. Obviously, a paper machine and "paper making" refers only to the latter one. Refiners of different types are used for grinding or milling wood chips to wood fibers for subsequent processing to a cellulose product, and for shredding, beating or refining pulp to individual fibers for papermaking. The more specific context of the text determines which one is meant.

US 2010/0121473 A discloses a system and method for optimizing a process for refining lignocellulosic granular matter such as wood chips which use a predictive model including a simulation model based on relations involving a plurality of matter properties characterizing the matter such as moisture content, density, light reflection or granular matter size, refining process operating parameters such as transfer screw speed, dilution flow, hydraulic pressure, plate gaps, or retention delays, at least one output controlled to a target such as primary motor load or pulp freeness, and at least one uncontrolled output such as specific energy consumption, energy split, long fibers, fines and shives. An adaptor is fed with measured values of matter properties and measured values of controlled and uncontrolled outputs, to adapt the simulation model accordingly. An optimizer generates a value of the target according to a predetermined condition on a predicted uncontrolled output parameter and to one or more process constraints.

### Summary of the Invention

A reliable and product-batch -specific quality index allows for more specific process control than what has been previously possible. With given process parameters, the CSF value of the pulp is more precisely predictable from the outset with a quality index at hand. This minimizes the need for costly trial runs and tests. A quality index that is representative of the Kappa number, which indicates the level of bleaching of the pulp, and/or the yield of the pulp, which corresponds to the expected tensile strength of the final paper product, allows a much improved process control in a papermaking factory.
The object of the present invention is therefore to create a new way of controlling a pulp mill, and refiner, where the correlation between a quality index and central processing parameters is established and is used in improved methods for setting and controlling the operation parameters of such a mill.
As will be later on demonstrated, it has been established and proved that a yield function-based quality index significantly corresponds with the quality of refined pulp. Other remaining major variables are due to the milling properties and other circumstances, like measurement variation and water quality. This leaves the paper manufacturer with a lot more information and predictability of the outcome than with conventional pulp refining methods and systems.

More specifically, the method according to the invention is defined in claim 1. Thus, the invention relates to a method for controlling a refiner for refining a product containing cellulose, such as pulp, and readable information about a quality index of the product, said method comprising the steps of:
- reading the quality index specific to said product;
- relating said quality index value to at least one predefined property of a slurry containing said product or of a finished product made of said slurry, that is proportional to said quality index;
- generating control signals in response to said quality index and a desired value of said property; and
- controlling in response to said control signals at least one operating parameter of said refiner for refining said product to correspond to said desired value of said predefined property, which property is being chosen from the group of the tensile strength of said finished product, and the Canadian Standard Freeness (CSF) of said slurry.

The quality index is representative of the received yield of bleached cellulose versus the amount of wood consumed in its preparation, and is based on an on-line measurement of product and process parameters in at least a cooking step, an oxygen treatment step and a bleaching process step, which results are normalized as contributions to said yield function being indicative of the amount of bleached cellulose received from the wood.

The quality of the pulp is first determined on basis of the wood raw material (or other plant material) used. The quality can be controlled by controlling portions of different raw material chips used for producing the pulp. One example of a usable mix is 30% pine roundwood, 20% spruce roundwood and 50% chips from saw mill residue.

Second factor determining quality is the process conditions at the pulp mill. This means that the yield graph of a pulping process can be effected by the process conditions at the mill. If the chemical dosage is high, the graph sets lower causing decrease in yield and strength of fibers. This causes a decrease of quality index. The model is based on theoretical knowledge and data-analysis of the process. These determine the quality index. When this quality index is taken into account, the refining step at the paper mill can be optimized.

The quality index depicts the process conditions at the pulp factory (pH, temperature, dosage of chemicals...), which have exactly determined effect on the quality of the pulp. Therefore the quality index combines several process conditions. A good quality and properties of fibers are considered to be obtained by gentle processing that is depicted as quality index.

At the pulp mills the length of the fibers in pulp is tried to be kept constant by using a same chips mix, allowing only small variation. The length of the fibers may be included in the information embedded in the pulp product, but the quality index depicts the chemical composition of the pulp and the strength of the fiber

The controllable operating parameter of the refiner may be its specific energy consumption, or any other variable that controls the performance of the equipment. Typically the refiner is controlled to keep the CSF value of the slurry produced on a desired level, or to obtain a desired tensile strength of a finished paper product produced. Additionally, by using a pulp dosage control loop, it is possible to obtain a constant CSF of the slurry and a constant tensile strength value of the finished paper product produced by a mill provided with the inventive refiner control system.
The control signaling made possible by the invention may be used as feed-forward control signals for the refiner, providing more precise and initially better control of it, in addition to conventional control signals from a feedback loop measuring the refined product. In an advantageous embodiment, the quality index information is provided on a machine readable medium attached to the cellulose bulk product in the form of a remotely readable RFID tag.

The system according to the invention is defined in claim 10. Thus, the invention also relates to a system for controlling a refiner for refining a product containing cellulose, such as pulp, and readable information about a quality index of the product, said system comprising:
- reading means for reading the quality index specific to said product;
- computing means for storing the values of at least one predefined property of a slurry containing said product or of a finished product made of said slurry, that is proportional to said quality index, and for generating control signals for at least one operation parameter of the refiner in response to said quality index and a desired value of said property; and
- control means being responsive to said control signal adapting said refiner to refine said product to correspond to the desired value of said predefined property, which is chosen from the group of the tensile strength of said finished product, and the Canadian Standard Freeness (CSF) of said slurry.
The product fed to a mill that hosts a refiner equipment being controlled by the inventive system, may be a bulk product, like a pulp unit. Each batch of the pulp units or each unit individually may have the quality index information provided on a passive RFID tag. Advantageously, the tag is formed on re-pulpable paper and fastened to the pulp unit with a water-soluble adhesive.

As has been explained, considerable advantages are obtained with the present invention. Next, the invention will be examined more closely with the aid of a detailed description of preferred embodiments.

### Brief Description of the Drawings

Figure 1 shows a diagram over the yield function and how process steps contribute to it;
Figure 2 is a graph over the CSF value as a function of a cellulose quality index;
Figure 3 shows a control system in a paper mill according to prior art;
Figure 4 shows an embodiment of a control system in a paper mill according to the present invention;
Figure 5 shows another embodiment of a control system in a paper mill according to the present invention.

### Description of Preferred Embodiments

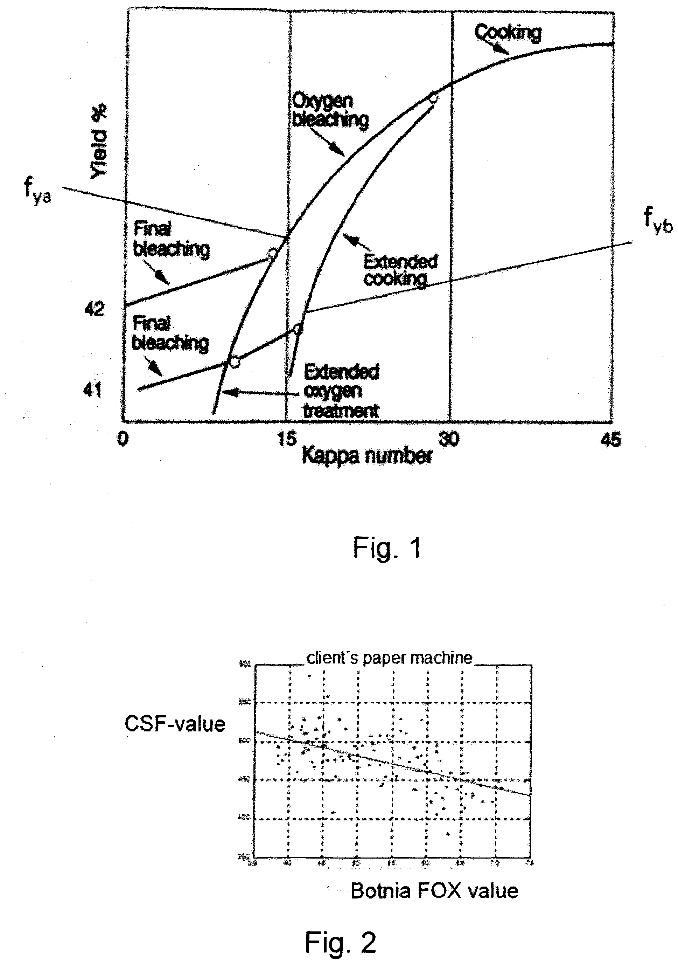

The present technology relates to a method and system using product quality information in order to control a refiner, for refining a product containing cellulose, such as pulp. Referring now to Fig. 1, a yield function is depicted, wherein the yield (%) is presented as a function of the Kappa number of the pulp. The Kappa number (having a value in the range of 1 to 100) is defined as an estimate of the amount of lignin.

In Fig. 1, the effect on the yield function by the various processes is clearly visible. The more processing and bleaching, the lower the yield will be. To achieve a certain bleach grade or Kappa number, processing has to continue until a reasonable value is achieved. It is also clear that the higher the yield, the more gentle the fiber treatment has been in the process, and the higher the tensile strength of the web in the paper machine will be.

Three process steps as shown on the yield function f_{ya}, are monitored to create a quality index of the final product: cooking, oxygen bleaching or treatment, and final bleaching. As can be seen from the figure, the oxygen step can be omitted (yield function f_{yb}), but then the yield will typically be lower if the Kappa number of the pulp fed to bleaching is maintained on the same level as when using an oxygen stage. On the other hand, if the oxygen delignification step is omitted without other changes made to the process conditions, the yield will be high.

Different process parameters have an impact on yield. Thus, yield is affected by temperature, pH, the chemicals added, process delays and other process parameters, chemicals and results to be followed. In the present technology, the parameters are normalized and combined, in one embodiment incrementally summed as such, to provide a global yield function, which is a quality index of the process. The combination can also comprise an incremental summing of the parameters after adjusting their values using modification coefficients. The calculation algorithm is selected by mathematical methods such that it corresponds to overall client feedback as well as possible. An example of such a quality index is the Botnia FOX™ index, which is a trademark of Metsä Fibre Oy, Finland.

The total yield function or quality index is of utmost importance, as it serves as a normalization factor, making it possible to add the individual yield functions of all steps incrementally to the total yield function. When the whole process and it steps can be described with one yield graph, it is possible to build a quality index algorithm having as its input basic knowledge and analysis results on one hand, and on-line process information (the yield function) on the other hand, see Fig. 1.

It should be pointed out that within the scope of the present novel technology it is possible to utilize the yield function concept also for other purposes, such as for cost calculations of the pulping process. Thus, the present concepts provides for minimization of production costs at pulp mill while attaining target product quality at paper or board mill.

Another use of the present concept is for minimizing production costs at paper mill.

Fig. 2 shows an empirically established graph over the CSF value in a paper machine as a function of the yield -based pulp quality index Botnia FOX™ (while the specific energy consumption of the mill was kept constant). Fig. 2 proves that a quality index, like the Botnia FOX™ index, of refined pulp is affecting the performance of the pulp in a papermaking process. This is the first time it has been proved statistically and surprisingly the influence of the pulp quality is bigger than anticipated. The Botnia FOX™ quality index correlation with the properties of the pulp has been verified during a period of 3 years at a paper machine. Information provided by an appropriate quality index is thus proven to be usable at a paper machine for process and quality control of the papermaking process. If the paper mill is refining pulp with a constant energy consumption, a low FOX™-value leads to a high CSF value, meaning refining of the pulp should be prolonged or increased. Or vice versa. Thus, for the first time, a paper mill can set its operating parameters for a new batch of pulp directly based on information available before the process starts, and expect a predictable outcome in terms of paper quality and properties.

The Botnia FOX™ index is provided on a small RFID tag on the market embedded in the pulp bales or units without affecting the product or its processing in any way. The RFID microchip may be of the size 0.6 x 0.6 x 0.1 mm, e.g. the size of a single grain of sand. The antenna may be made of silver paste, and the tag is fastened with water-soluble adhesive to the pulp bale or unit. It has been found that such tags entirely disperse at the pulping stage. In relation to the huge scale of the paper manufacturing process, a single tiny tag in each tonne of pulp is considered a negligible foreign body in the resulting paper or end product. RFID tags are becoming even smaller, thinner and less complex while retaining excellent readability. The RFID tags are fastened to the pulp units near enough to the outer surface of the pulp unit to enable efficient reading, but sufficiently embedded to ensure that they remain undamaged. There is no need for a direct line of sight between the reader and the tag, and several units can be identified with a single reading sweep. As the tag is inside the pulp unit, its exposure to soiling, physical stresses and other external disturbances is minimised.

The quality index implemented on an RFID tag will also help to optimise warehouse management, improving the efficiency of labour and automating functions that were previously performed manually. All tracking information is available in real-time. This brings benefits for the supply chain, including logistical partners and customers by providing continuous real-time information on warehouse and raw material levels and on pulp quality and highly automated tracking of products as they move around the world.

### Examples

### Example 1.

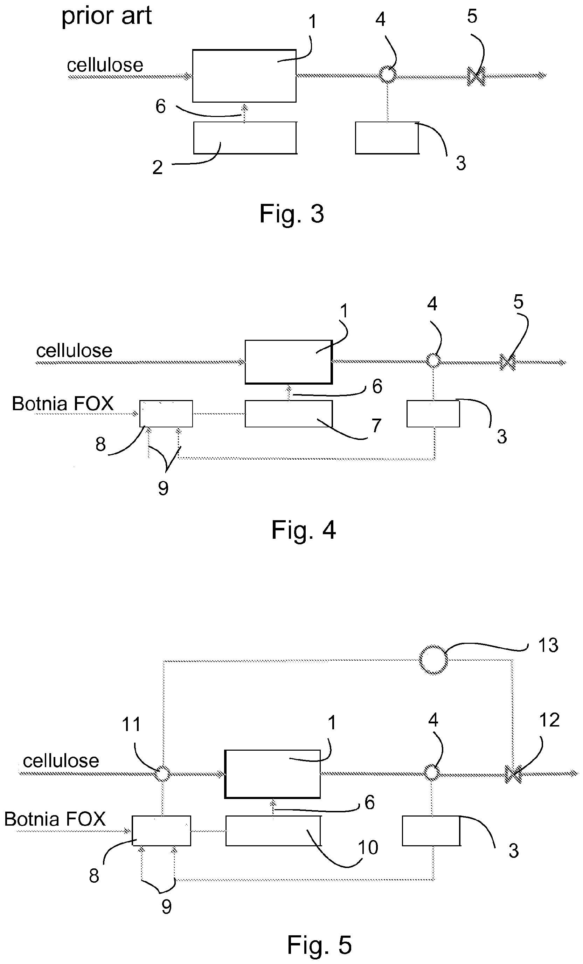

In fig. 3 is shown a control system in a paper mill according to prior art, where pulp is refined with a refiner having a constant specific energy consumption. The process control parameters may include the refining consistency and flow properties of the slurry and the specific edge load at the blades. However, these parameters do not give a statistically reliable indication or prediction of the CSF- value of refined pulp. The main components of the system shown are the pulp input, the refiner 1, the process control system 2 controlling the specifig energy consumption of the refiner by a signal 6, a CSF measurement unit 3,4 and a pulp slurry outlet controlled by a valve 5.

CSF values are read at unit 3 and the process control system 2 is adjusted accordingly to this feedback to make the milling in the refiner 1 more or less effective.

### Example 2.

In Fig. 4 is shown a control system in a paper mill using the present invention. Like in Fig. 3, there is feedback provided from a CSF measurement unit 3,4 and from conventional process control parameters as described above, depicted as input signals 9 to a milling modeling unit 8. Here also the quality index Botnia FOX™ is provided as an input, to serve as a basis for calculating a milling profile for the pulp to be fed into the control system 7. This creates a feed-forward loop 8->7->1 that enhances the control quality and speed of the process. The delay in the control system using the predefined quality index information of the pulp is smaller than the delay in the CSF feedback control loop 4->3->8->7->1. In practice, this means that if a process parameter changes, including the quality index value , it is possible to make a correction to the refiner operation at 6, even before the CSF value at 4 changes to an undesired value.

The inventive approach using a pulp quality index, like the Botnia FOX™ index opens up new perpectives in the art of controlling paper factory milling. The milling control in a paper machine can take place in three different ways. The papermaker can choose between milling with:
- a constant CSF value,
- a constant tensile strength, or
- a constant CSF and tensile strength value (see Fig. 5)

### Example 3.

In Fig 5 is shown another embodiment of the invention, where is added a pulp dosage control loop at 11, 12, 13. This enables one to maintain at the same time both a constant CSF and tensile strength value of the product, i.e. the paper. The CSF is controlled as in Fig. 4. If the flow meter 13 that is measuring the slurry flow rate at valve 12 receives an indication that the quality index or some other process parameter has changed, the celloslose dosing at 11 can be changed to ensure that the desired tensile strength or stiffness is maintained and achieved. It is to be understood that this control loop is made possible by the added predictability of the process provided by a pulp quality index and by the inventive insight that such an index may a provide a statistically significant and stable indication on the refining properties

## Claims

1. A method for controlling a refiner for refining a product containing cellulose, such as pulp, and readable information about a quality index of the product, said method comprising the steps of:
- reading the quality index specific to the product that is refined;
- relating said quality index value to at least one predefined property of a slurry containing said product or of a finished product made of said slurry, that is proportional to said quality index;
- generating control signals in response to said quality index and a desired value of said property, and
- controlling in response to said control signals at least one operating parameter of said refiner for refining said product to correspond to said desired value of said predefined property, which property is being chosen from the group of the tensile strength of said finished product, and the Canadian Standard Freeness (CSF) of said slurry.

2. A method according to claim 1, **characterized in that** the quality index is representative of the received yield of bleached pulp versus the amount of wood consumed in the preparation of said cellulose product.

3. A method according to claim 2, **characterized in that** said quality index is based on on-line measurement of product and process parameters in a manufacturing process of said cellulose product in at least a cooking step, an oxygen treatment step and a bleaching process step, which results are normalized as contributions to a yield function being indicative of the amount of bleached pulp received from the wood.

4. A method according to any of the preceding claims, **characterized by** using as a controllable operating parameter the specific energy consumption of said refiner.

5. A method according to any of the preceding claims, **characterized by** using said control signals to obtain a desired and constant CSF value of the slurry produced by the refiner.

6. A method according to any of the preceding claims, **characterized by** using said control signals to obtain a desired tensile strength of the final paper product produced by a mill using said refiner.

7. A method according to any of the preceding claims, **characterized by** using said control signals and a pulp dosage control loop to obtain a desired and constant CSF of the slurry at the refiner and a desired tensile strength value of the final paper product produced by a mill using said refiner.

8. A method according to any of the preceding claims, **characterized by** using said control signals as feed-forward control signals for said refiner in addition to second control signals obtained from a feedback loop measuring the refined product.

9. A method according to any of the preceding claims, **characterized by** that the quality index information is provided on a machine readable medium attached to said product in the form of a remotely readable RFID tag.

10. A system for controlling a refiner for refining a product containing cellulose, such as pulp, and readable information about a quality index of the product, said system comprising:
- reading means for reading the quality index specific to said product to be refined;
- computing means for storing the values of at least one predefined property of a slurry containing said product or of a finished product made of said slurry, that is proportional to said quality index, and for generating control signals for at least one operation parameter of the refiner in response to said quality index and a desired value of said property, and
- control means being responsive to said control signal adapting said refiner to refine said product to correspond to the desired value of said predefined property, which is chosen from the group of the tensile strength of said finished product, and the Canadian Standard Freeness (CSF) of said slurry.

11. A system according to claim 10, **characterized in that** an operating parameter of the refiner or grinder that is responsive to said control signals is the specific energy consumption of said refiner.

12. A system according to claim 10 or 11, **characterized in that** the control signals are used to obtain a constant CSF value of the slurry produced by the refiner.

13. A system according to claim 10 or 11, **characterized in that** the control signals are used to obtain a constant tensile strength of the final paper product produced by a mill using said refiner.

14. A system according to any of claims 10 to 13, **characterized in that** said control signals and a pulp dosage control loop is used to obtain a constant CSF of the slurry at the refiner and a constant tensile strength value of the final paper product produced by a mill using said refiner.

15. A system according to any of claims 10 to 14, **characterized in that** said control signals are used as a feed-forward control signals for said refiner in addition to second control signals from a feedback loop measuring the refined product.

16. A system according to any of claims 10 to 15, **characterized in that** said product is a bulk product like a pulp unit having a quality index corresponding to a yield function that is based on on-line measurement of product and individual process parameters in a manufacturing process of said cellulose product, selected for example from cooking, oxygen treatment and bleaching process steps, whereby the results are calculated and normalized as contributions to said yield function being indicative of the amount of bleached pulp received from the wood.

17. A system according to any of claims 10 to 16, **characterized in that** the quality index information is provided on a passive RFID tag attached to said product.

18. A system according to claim 17, **characterized in that** said RFID tag is formed on re-pulpable paper and fastened with a water-soluble adhesive to a unit of said product.

## Patentansprüche

1. Verfahren zum Steuern eines Refiners zum Mahlen eines Cellulose enthaltenden Produkts, wie Zellstoff, und lesbarer Informationen über einen Qualitätsindex des Produkts, wobei das Verfahren die Schritte umfasst:
- Lesen des Qualitätsindexes, der für das zu vermahlende Produkt spezifisch ist;
- Verknüpfen des Qualitätsindexwertes mit mindestens einer vordefinierten Eigenschaft einer das Produkt enthaltenden Aufschlämmung oder eines aus der Aufschlämmung hergestellten Fertigprodukts, die proportional zum Qualitätsindex ist;
- Erzeugen von Steuersignalen als Reaktion auf den Qualitätsindex und einen Sollwert der Eigenschaft, und
- Steuern mindestens eines Betriebsparameters des Refiners zum Mahlen des Produkts als Reaktion auf die Steuersignale, um dem Sollwert der vordefinierten Eigenschaft zu entsprechen, wobei die Eigenschaft aus der Gruppe der Zugfestigkeit des Fertigprodukts und der Kanadischen Standardfeinheit (CSF) der Aufschlämmung ausgewählt wird.

2. Verfahren nach Anspruch 1, **dadurch gekennzeichnet, dass** der Qualitätsindex repräsentativ für die erhaltene Ausbeute an gebleichtem Zellstoff im Vergleich zur Menge an Holz ist, die bei der Herstellung des Celluloseprodukts verbraucht wird.

3. Verfahren nach Anspruch 2, **dadurch gekennzeichnet, dass** der Qualitätsindex auf einer Online-Messung von Produkt- und Verfahrensparametern in einem Herstellungsverfahren des Zelluloseprodukts in mindestens einem Kochschritt, einem Sauerstoffbehandlungsschritt und einem Bleichverfahrensschritt basiert, wobei die Ergebnisse als Beiträge zu einer Ausbeutefunktion normiert werden, die die Menge an gebleichtem Zellstoff anzeigt, die vom Holz aufgenommen wird.

4. Verfahren nach einem der vorstehenden Ansprüche, **gekennzeichnet durch** die Verwendung des spezifischen Energieverbrauchs des Refiners als steuerbarer Betriebsparameter.

5. Verfahren nach einem der vorstehenden Ansprüche, **gekennzeichnet durch** die Verwendung der Steuersignale, um einen gewünschten und konstanten CSF-Wert der durch den Refiner erzeugten Aufschlämmung zu erhalten.

6. Verfahren nach einem der vorstehenden Ansprüche, **gekennzeichnet durch** die Verwendung der Steuersignale, um eine gewünschte Zugfestigkeit des von einer Anlage unter Verwendung des Refiners hergestellten Papierfertigprodukts zu erhalten.

7. Verfahren nach einem der vorstehenden Ansprüche, **gekennzeichnet durch** die Verwendung der Steuersignale und eines Zellstoffdosierungsregelkreises, um ein gewünschtes und konstantes CSF der Aufschlämmung am Refiner und einen Zugfestigkeitssollwert des von einer Anlage unter Verwendung des Refiners hergestellten Papierfertigprodukts zu erhalten.

8. Verfahren nach einem der vorstehenden Ansprüche, **gekennzeichnet durch** die Verwendung der Steuersignale als Feed-Forward-Steuersignale für den Refiner zusätzlich zu zweiten Steuersignalen, die aus einer Rückkopplungsschleife zum Messen des veredelten Produkts erhalten werden.

9. Verfahren nach einem der vorstehenden Ansprüche, **dadurch gekennzeichnet, dass** die Qualitätsindexinformationen auf einem maschinenlesbaren Medium bereitgestellt werden, das an dem Produkt in Form eines fernlesbaren RFID-Tags befestigt ist.

10. System zum Steuern eines Refiners zum Mahlen eines Produkts, das Cellulose, wie beispielsweise Zellstoff, und lesbare Informationen über einen Qualitätsindex des Produkts enthält, wobei das System umfasst:
- Lesemittel zum Lesen des für das zu vermahlende Produkt spezifischen Qualitätsindexes;
- Berechnungsmittel zum Speichern der Werte mindestens einer vordefinierten Eigenschaft einer das Produkt enthaltenden Aufschlämmung oder eines aus der Aufschlämmung hergestellten Fertigprodukts, die proportional zum Qualitätsindex ist, und zum Erzeugen von Steuersignalen für mindestens einen Betriebsparameter des Refiners als Reaktion auf den Qualitätsindex und einen Sollwert der Eigenschaft, und
- Steuermittel, die auf das Steuersignal reagieren unter Anpassen des Refiners, um das Produkt zu vermahlen, um dem Sollwert der vordefinierten Eigenschaft zu entsprechen, die aus der Gruppe der Zugfestigkeit des Fertigprodukts und der Kanadischen Standardfeinheit (CSF) der Aufschlämmung ausgewählt ist.

11. System nach Anspruch 10, **dadurch gekennzeichnet, dass** ein Betriebsparameter des Refiners oder des Schleifers, der auf die Steuersignale reagiert, der spezifische Energieverbrauch des Refiners ist.

12. System nach Anspruch 10 oder 11, **dadurch gekennzeichnet, dass** die Steuersignale verwendet werden, um einen konstanten CSF-Wert der durch den Refiner erzeugten Aufschlämmung zu erhalten.

13. System nach Anspruch 10 oder 11, **dadurch gekennzeichnet, dass** die Steuersignale verwendet werden, um eine konstante Zugfestigkeit des von einer Anlage unter Verwendung des Refiners hergestellten Papierfertigprodukts zu erhalten.

14. System nach einem der Ansprüche 10 bis 13, **dadurch gekennzeichnet, dass** die Steuersignale und ein Zellstoffdosierungsregelkreis verwendet werden, um ein konstantes CSF der Aufschlämmung am Refiner und einen konstanten Zugfestigkeitswert des von einer Anlage unter Verwendung des Refiners hergestellten Papierfertigprodukts zu erhalten.

15. System nach einem der Ansprüche 10 bis 14, **dadurch gekennzeichnet, dass** die Steuersignale als Feed-Forward-Steuersignale für den Refiner zusätzlich zu zweiten Steuersignalen aus einem Rückführkreis, der das vermahlene Produkt misst, verwendet werden.

16. System nach einem der Ansprüche 10 bis 15, **dadurch gekennzeichnet, dass** das Produkt ein Massenprodukt, wie eine Zellstoffeinheit mit einem Qualitätsindex ist, der einer Ausbeutefunktion entspricht, die auf einer Online-Messung des Produkts und einzelner Verfahrensparameter in einem Herstellungsverfahren des Zelluloseprodukts basiert, ausgewählt beispielsweise aus den Verfahrensschritten Kochen, Sauerstoffbehandlung und Bleichen, wobei die Ergebnisse als Beiträge zu der Ausbeutefunktion berechnet und normiert werden, die die Menge des vom Holz aufgenommenen gebleichten Zellstoffs anzeigen.

17. System nach einem der Ansprüche 10 bis 16, **dadurch gekennzeichnet, dass** die Qualitätsindexinformationen auf einem passiven RFID-Tag bereitgestellt werden, der an dem Produkt angebracht ist.

18. System nach Anspruch 17, **dadurch gekennzeichnet, dass** der RFID-Tag auf wiederauflösbarem Papier gebildet und mit einem wasserlöslichen Klebstoff an einer Einheit des Produkts befestigt ist.

## Revendications

1. Procédé pour commander un raffineur pour raffiner un produit contenant de la cellulose, tel qu'une pâte, et des informations lisibles sur un indice de qualité du produit, ledit procédé comprenant les étapes de :
- lire l'indice de qualité spécifique au produit raffiné ;
- relier ladite valeur d'indice de qualité à au moins une propriété prédéfinie d'une boue contenant ledit produit ou d'un produit fini constitué de ladite boue, proportionnelle audit indice de qualité ;
- générer des signaux de commande en réponse audit indice de qualité et à une valeur souhaitée de ladite propriété ; et
- commander en réponse auxdits signaux de commande au moins un paramètre de fonctionnement dudit raffineur pour raffiner ledit produit pour correspondre à ladite valeur désirée de ladite propriété prédéfinie, ladite propriété est choisie dans le groupe comprenant la résistance à la traction dudit produit fini et l'indice d'égouttage canadien standard (Canadian Standard Freeness (CSF)) de ladite boue.

2. Procédé selon la revendication 1, **caractérisé en ce que** l'indice de qualité est représentatif du rendement reçu en pâte blanchie par rapport à la quantité de bois consommée dans la préparation dudit produit cellulosique.

3. Procédé selon la revendication 2, **caractérisé en ce que** ledit indice de qualité est basé sur une mesure en ligne de paramètres de produit et de procédé dans un procédé de fabrication dudit produit cellulosique dans au moins une étape de cuisson, une étape de traitement à l'oxygène et une étape de procédé de blanchiment, dont les résultats sont normalisés en tant que contributions à une fonction de rendement indicative de la quantité de pâte blanchie reçue du bois.

4. Procédé selon l'une quelconque des revendications précédentes, **caractérisé par** l'utilisation comme paramètre de fonctionnement pouvant être commandé de la consommation d'énergie spécifique dudit raffineur.

5. Procédé selon l'une quelconque des revendications précédentes, **caractérisé en ce que** l'on utilise lesdits signaux de commande pour obtenir une valeur de CSF souhaitée et constante de la boue produite par le raffineur.

6. Procédé selon l'une quelconque des revendications précédentes, **caractérisé par** l'utilisation desdits signaux de commande pour obtenir une résistance à la traction souhaitée du produit final en papier produit par une usine utilisant ledit raffineur.

7. Procédé selon l'une quelconque des revendications précédentes, **caractérisé par** l'utilisation desdits signaux de commande et d'une boucle de commande de dosage de pâte pour obtenir un CSF souhaité et constant de la boue au niveau du raffineur et une valeur de résistance à la traction souhaitée du produit final en papier produit par une usine utilisant ledit raffineur.

8. Procédé selon l'une quelconque des revendications précédentes, **caractérisé par** l'utilisation desdits signaux de commande en tant que signaux de commande à action directe pour ledit raffineur en plus de seconds signaux de commande obtenus à partir d'une boucle de rétroaction mesurant le produit raffiné.

9. Procédé selon l'une quelconque des revendications précédentes, **caractérisé en ce que** les informations d'indice de qualité sont fournies sur un support lisible par machine attaché audit produit sous la forme d'une étiquette RFID lisible à distance.

10. Système de commande d'un raffineur pour raffiner un produit contenant de la cellulose, tel qu'une pâte, et d'informations lisibles sur un indice de qualité du produit, ledit système comprenant :
- des moyens de lecture pour lire l'indice de qualité spécifique audit produit à raffiner ;
- des moyens informatiques pour stocker les valeurs d'au moins une propriété prédéfinie d'une boue contenant ledit produit ou d'un produit fini constitué de ladite boue, qui est proportionnelle audit indice de qualité, et pour générer des signaux de commande pour au moins un paramètre de fonctionnement du raffineur en réponse audit indice de qualité et à une valeur souhaitée de ladite propriété ; et
- un moyen de commande sensible audit signal de commande adaptant ledit raffineur pour raffiner ledit produit afin qu'il corresponde à la valeur désirée de ladite propriété prédéfinie, qui est choisie dans le groupe comprenant la résistance à la traction dudit produit fini et l'indice d'égouttage canadien standard (Canadian Standard Freeness (CSF) de ladite boue.

11. Système selon la revendication 10, **caractérisé en ce qu'**un paramètre de fonctionnement du raffineur ou broyeur sensible auxdits signaux de commande est la consommation d'énergie spécifique dudit raffineur.

12. Système selon la revendication 10 ou 11, **caractérisé en ce que** les signaux de commande sont utilisés pour obtenir une valeur CSF constante de la boue produite par le raffineur.

13. Système selon la revendication 10 ou 11, **caractérisé en ce que** les signaux de commande sont utilisés pour obtenir une résistance à la traction constante du produit final en papier produit par une usine utilisant ledit raffineur.

14. Système selon l'une quelconque des revendications 10 à 13, **caractérisé en ce que** lesdits signaux de commande et une boucle de commande de dosage de pâte sont utilisés pour obtenir un CSF constant de la boue au niveau du raffineur et une valeur de résistance constante du produit final en papier produit par une usine utilisant ledit raffineur.

15. Système selon l'une quelconque des revendications 10 à 14, **caractérisé en ce que** lesdits signaux de commande sont utilisés en tant que signaux de commande à action directe pour ledit raffineur en plus de seconds signaux de commande provenant d'une boucle de rétroaction mesurant le produit raffiné.

16. Système selon l'une quelconque des revendications 10 à 15, **caractérisé en ce que** ledit produit est un produit en vrac tel qu'une unité de pâte ayant un indice de qualité correspondant à une fonction de rendement qui est basée sur une mesure en ligne de paramètres de produit et de procédé individuel dans un procédé de fabrication dudit produit cellulosique, choisi par exemple à partir d'étapes de cuisson, de traitement à l'oxygène et de procédé de blanchiment, de sorte que les résultats sont calculés et normalisés en tant que contributions à ladite fonction de rendement qui est indicative de la quantité de pâte blanchie reçue du bois.

17. Système selon l'une quelconque des revendications 10 à 16, **caractérisé en ce que** les informations d'indice de qualité sont fournies sur une étiquette RFID passive attachée audit produit.

18. Système selon la revendication 17, **caractérisé en ce que** ladite étiquette RFID est formée sur du papier re-pulpable et fixée avec un adhésif soluble dans l'eau à une unité dudit produit.
